# EUROPEAN PATENT SPECIFICATION

(11) **EP 2 671 601 B1**
(45) Date of publication and mention of the grant of the patent: **16.03.2016**
(21) Application number: 12171154.3
(22) Date of filing: 07.06.2012
(51) Int. Cl.: A61M 3/02

(54) **Irrigation system with pre-loaded irrigation liquid reservoir**
Irrigationssystem mit vorbeladenem Irrigationsflüssigkeitsbehälter
Système d'irrigation avec réservoir de liquides d'irrigation pré-chargé

(43) Date of publication of application: 11.12.2013
(73) Proprietor: Dentsply IH AB, 431 21 Mölndal (SE)
(72) Inventor: Andréen, Erik, 417 57 Göteborg (SE); Andersson, Fredrik, 411 25 Göteborg (SE)
(74) Representative: Awapatent AB

(56) References cited:
- WO-A1-98/23312
- GB-A- 238 038
- US-A- 1 473 979
- US-A- 5 498 237
- US-A1- 2003 073 963
- US-B2- 7 150 739

## Description

### Technical Field

The present invention relates to an irrigation system, comprising a reservoir for an irrigation liquid, a probe for arrangement in a user, and a control unit. The irrigation system is particularly intended for rectal irrigation, and is suitable for self-administration of an irrigation liquid.

### Background of the Invention

The present invention relates to an irrigation device, see e.g. US2003073963. Administrating an irrigation liquid is a common medical procedure whereby liquid is injected into a bodily cavity, such as into the rectum and lower intestine of a patient in order to induce bowel movement. The need for such a procedure typically arises in patients suffering from certain physical ailments in which voluntary bowel control is impaired or when the bowel needs to be cleaned before e.g. a coloscopy or a surgical operation. To this end, irrigation systems may be used e.g. by people suffering from spinal cord injuries, spina bifida or multiple sclerosis. For such users, irrigation may improve quality of life by preventing constipation, reducing time spent for bowel emptying procedures, reducing fecal incontinence, and by increasing independency in general.

Irrigation is nowadays often performed outside medical attendance premises, such as in the patient's home, and is also often performed by the patient himself, i.e. by self-administration. Hereby, the patient need to do multiple tasks at the same time, or immediately following on each other, such as inserting the probe in a correct position, adequately fixating the probe in the bodily cavity, enabling the liquid to be discharged for irrigation and discharge a correct dose of irrigation liquid, and removing the probe after use. Further, many of the users of irrigation systems have reduced dexterity, which makes the operation even more cumbersome.

It is further of importance that the irrigation system is of a limited size, and portable. Portability of the irrigation system is important to disabled persons who are not hospitalised or bed-ridden if they are to live as normal a life as possible. This is particularly important if they travel away from their home, for instance, to someone else's home or if they stay in a hotel. In this situation, they need to be able to deal with their bowel function easily.

Various irrigation systems are known in the art, such as is disclosed in WO 2008/087220, WO 2009/092380, WO 03/030969, WO 2011/023196 and WO 03/030968. However, despite the attempts to make these devices user friendly, all of these irrigation devices are still relatively complicated to use, especially for self-administration of the irrigation liquid, and also, most of these known devices are relatively large and relatively costly to produce.

There is therefore a need for an irrigation device which can be used safely, easily and conveniently for self-administration of the irrigation liquid, and which can be produced in a cost-efficient way.

### Summary of the Invention

In view of the above mentioned need, a general object of the present invention is to provide an irrigation system which alleviates the above-discussed problems of the prior art, and at least partly fulfils the above-discussed needs.

This and other objects are achieved with an irrigation system according to the appended claims.

According to the invention, there is provided an irrigation system comprising:
a reservoir for an irrigating liquid;
a probe for arrangement in a user;
a control unit for controlling the transfer of said irrigation liquid;
tubing providing fluid communication between said reservoir, control unit and probe;
wherein said reservoir is arranged to exert a mechanical pressure on the irrigation liquid therein, thereby maintaining the irrigation liquid under pressure, and wherein said control unit comprises a control valve for releasing irrigation liquid from said reservoir for discharge through said probe.

The irrigation system of the present invention comprises relatively few and uncomplicated components, which makes the irrigation system relatively easy and cost-efficient to produce. Further, the irrigation system lends itself well for automated or semi-automated manufacturing.

Further, the irrigation system of the present invention is very safe and easy to use, also for persons with reduced dexterity. This also makes the irrigation system highly suitable for self-administration of the irrigation liquid. Since the reservoir is arranged to exert a mechanical pressure on the irrigation liquid therein, it maintains the irrigation liquid under pressure. This pressure then enables the irrigation liquid to be transferred to the probe without any pumping action. The user simply operates the control valve for releasing irrigation liquid from the reservoir for discharge through the probe. The control valve can e.g. be operated by turning a control knob or depressing a control button. Such an operation is easy to perform even for persons with reduced dexterity, and requires no significant force. The operation of the control valve may e.g. be done by means of a finger, an elbow, the mouth, etc, which provides great flexibility in how the irrigation system can be used, and also enables the user to do other tasks during, or immediately before or after, such as controlling that the probe is kept correctly in place, etc.

The pumping required by prior art solutions is here replaced by a pre-loading of the reservoir to provide a pressurized liquid supply. This can e.g. be accomplished by mechanical tensioning of springs and the like. However, preferably, the reservoir is arranged to be loaded by means of the hydraulic pressure in common water distribution systems. Hereby, it is sufficient simply to connect the reservoir to a water tap or the like and turn on the water to load the reservoir. Hereby, loading in itself can be made simple. Further, since the pre-loading and pressurization of the reservoir can be performed prior to irrigation, when the user may focus solely on this task, the overall irrigation procedure is hereby greatly facilitated. Further, the reservoir may also be loaded a short or long time prior to the actual use.

According to one embodiment, the reservoir comprises an elastically stretchable container, which when elastically stretched exerts a mechanical pressure on the irrigation liquid therein. Preferably, the reservoir may hereby form an elastically stretchable balloon, which is inflated by the irrigation liquid, and thereby provides a pressure to expel the irrigation liquid when the control valve is opened. To prevent overfilling of the reservoir, the reservoir may further comprise an outer container of a non-stretchable material. Hereby, the outer container forms an outer boundary for the elastically stretchable container. The non-stretchable outer container may be formed as a rigid outer container, or as a flexible outer container. For example, a flexible outer container may be formed as a net of a non-stretchable material.

Further, an overload valve or pressure relief valve may be arranged to avoid overfilling of the reservoir. If tap water is used for filling, the overload valve may be arranged close to the connection to the tap/spigot. Hereby, release through the overload valve will automatically be released into the sink. However, additionally or alternatively, the overload valve may be arranged on the reservoir, or along the tube connecting the reservoir to the tap.

According to another embodiment, the reservoir is formed of a rigid or semi-rigid material, and having at least one moveable wall and a retention element to exert a force to retain the moveable wall in a contracted position. The retention element may be a spring, and preferably a coil spring. The spring may be arranged to exert a pushing force on the moveable wall, or to exert a pulling force. Further, many alternative retention elements are feasible, such as elastic cords, gas springs and the like.

The probe may comprise a fixing element, for fixing the probe in the inserted position for irrigation. Preferably, the probe comprises an inflatable balloon for fixing the catheter in a body cavity, and wherein the control unit is further arranged to control the fluid for said balloon.

The inflatable balloon may be inflated with any fluid, such as a gas or a liquid. According to one embodiment, the inflatable balloon is filled with the irrigation liquid. To this end, the control unit further preferably comprises a second control valve for releasing irrigation liquid from the reservoir for inflation of the inflatable balloon. According to another embodiment, the irrigation system may comprise a pump arranged for pumping a fluid, such as air, to the balloon for inflation. In this embodiment, the pump is preferably a manually operable pump, such as a bulb or a bellow pump. The control unit further preferably comprises a third control valve for releasing the fluid from the balloon when inflated, for deflation. The provision of a valve for releasing fluid from the balloon for deflation ensures that the balloon can be easily deflated after irrigation, and also that the filling level of the balloon can be precisely controlled, which makes it possible to adjust and optimize the filling level of the balloon during initial inflation, when the probe is fixed in the bodily cavity, or during irrigation.

A manually operable pump is very cost-efficient, which lowers the overall costs of the product. Further, a manually operable pump provides a very precise controllability for the user. Preferably, the manually operable pump is a bulb pump, comprising an inlet provided with a one-way valve, allowing a fluid to enter but not exit the pump, a pumping compartment and an outlet, provided with a one-way valve, allowing a fluid to exit but not enter the pump. The pumping compartment is made of a resilient, squeezable material, which retains it shape when unloaded. By squeezing the pumping compartment, the fluid is pumped out through the outlet, and when the squeezing is relieved, the pumping compartment retains its original shape, thereby sucking in fluid through the inlet.

When air is used to fill the balloon, the valve for releasing pressure may be a vent, discharging air out from the system for deflation. If e.g. irrigation liquid is used, the valve may instead release the irrigation liquid back to the reservoir or into the probe for discharge during irrigation. However, it is also possible to release the fluid from the balloon into a drain compartment or the like.

The irrigation system further preferably comprises a volume indicator arranged within the reservoir. This enables the user to precisely monitor and control the dose of irrigation liquid being used. According to one embodiment, the volume indicator comprises two telescopically connected parts, and a measuring element connected to one of said parts and extending out through the other of said parts. The telescopically connected parts preferably extend through the reservoir, and each part being connected to one wall of the reservoir. Hereby, when the distance between the walls changes due to filling of the reservoir, this is translated into a movement of the measuring element. Alternatively, the measuring scale or measuring indications may be arranged on the inner telescopic part, whereby any separate measuring element becomes redundant. This alternative is particularly useful when the reservoir is made wholly or partially of transparent material. The reading on the scale may be taken at the interjunction between the two telescopic parts. Alternatively, if also the outer telescopic part is at least partly transparent, a reading marker or the like may be arranged on the surface of the outer telescopic part. It is also possible to arrange the measuring scale or measuring indication on the outer telescopic part, and to have the marker on the inner telescopic part.

The telescopic parts may also be operatively connected to a release valve to prevent overload of the reservoir. Hereby, the valve may be opened when a certain extension position between the telescopic parts has been reached, and release liquid from the reservoir. The released liquid may e.g. be released back to a position near the filling input, by a return tube running parallel to the filling tube. However, alternative configurations for the release are also feasible.

The control unit may have a planar shape, with tubing extending therethrough. However, preferably, the control unit has a curved or angled shape.

According to another aspect, there is provided a use of the irrigation device as discussed above for rectal irrigation.

These and other aspects of the invention will be apparent from and elucidated with reference to the embodiments described hereinafter.

### Brief description of the drawings

For exemplifying purposes, the invention will be described in closer detail in the following with reference to embodiments thereof illustrated in the attached drawings, wherein:
Fig 1 is schematic overview of an irrigation system according to a first embodiment of the invention;
Fig 2 is a schematic overview of an irrigation system according to a second embodiment of the invention;
Fig 3 is a schematic overview of an irrigation system according to a third embodiment of the invention;
Fig 4 is a schematic overview of an irrigation system according to a fourth embodiment of the invention;
Figs 5A and 5B are a cross-sectional view and a perspective view of the reservoir of the embodiment in Fig 4;
Figs 6A and 6B are schematic overviews of control units having alternative shapes; and
Figs 7A and 7B are schematic overviews of reservoirs having alternative measuring means.

### Detailed description of preferred embodiments

The present invention will now be described more fully hereinafter with reference to the accompanying drawings, in which currently preferred embodiments of the invention are shown. This invention may, however, be embodied in many different forms and should not be construed as limited to the embodiments set forth herein; rather, these embodiments are provided for thoroughness and completeness, and fully convey the scope of the invention to the skilled addressee. Like reference characters refer to like elements throughout.

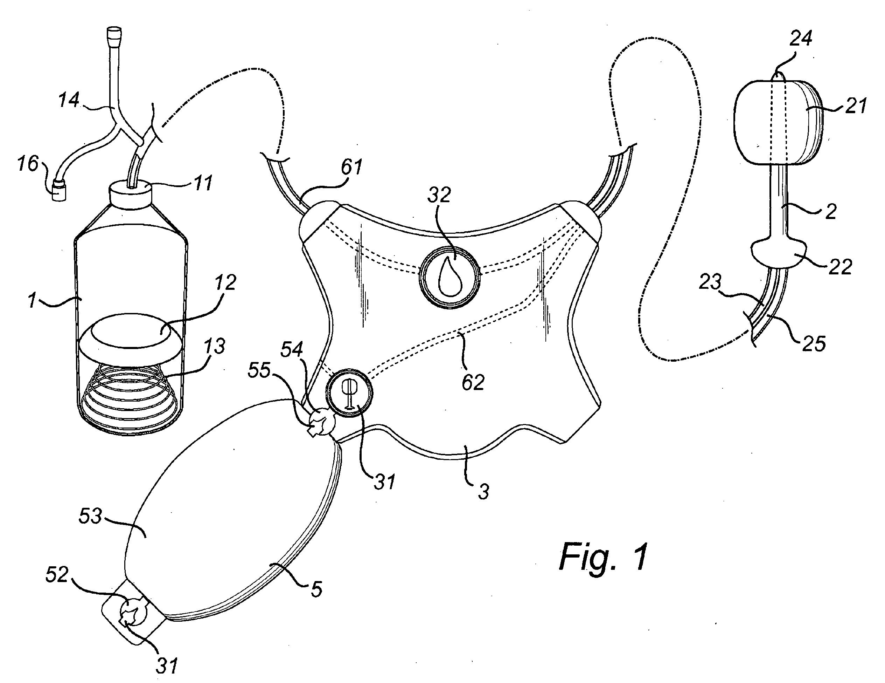

Fig 1 discloses an irrigation system according to a first exemplary embodiment, comprising a reservoir 1 for an irrigating liquid, a probe 2 for arrangement in a user, and a control unit 3.

The reservoir may be realized in various ways. For example, the reservoir may be formed by a rigid, semi-rigid or flexible material. In case a semi-rigid or flexible material is used, the reservoir may be collapsible or foldable, to make the irrigation system more compact prior to use. The reservoir is provided with an opening, closed by a lid 11, for filling of the reservoir. Tubing connecting the reservoir to the rest of the irrigation system may be provided through the lid 11, or through other access points on the reservoir.

In the first embodiment, the reservoir is preferably made of a rigid or semi-rigid material. A suitable material for the reservoir may be any suitable synthetic or natural polymeric material, metal, hard rubbery substances or other material having the desired rigidity. The reservoir in this example has a movable wall 12 arranged within the reservoir, to separate the reservoir into two sealed compartments. The movable wall 12 is further connected to a spring 13, here in the form of a coil spring, which exerts a pressure on the movable wall. Hereby, the reservoir is arranged to exert a mechanical pressure on the irrigation liquid therein, thereby maintaining the irrigation liquid under pressure.

The reservoir may comprise an additional inlet 14, which may be connected to a water tap or the like. Hereby, pre-loading of the reservoir may be achieved conveniently just by connecting the reservoir to the ordinary water distribution system. An overload valve or pressure relief valve 16 may be arranged close to the additional inlet 14, to allow water to flow into the.sink when a maximal filling has been obtained.

In order to render the irrigation system as portable as possible, the container preferably has a capacity of less than 5 litres, more preferred less than 3 litres and most preferred less than 2 litres. If however the system is to be used for repeated irrigation, a larger capacity container may be necessary.

The reservoir may comprise an overpressure release valve, to release pressure over a predetermined maximum pressure to be allowed.

The probe 2 may be provided with an insertable balloon 21 for fixing the catheter in a body cavity. Further, the probe may be provided with a rearward enlarged part 22, providing an abutment to hinder too deep insertion. The probe is hereby provided with two lumens - one lumen 23 for transfer of irrigation liquid through the probe, for discharge at the forward end 24, and one lumen 25 for inflation and deflation of the balloon.

The control unit is here realized as a plate. It further comprises, connected thereto, and preferably integrated in the control unit, a pump 5 for pumping a fluid to the balloon for inflation.

The control unit is further preferably provided with a valve for releasing fluid from the balloon for deflation, and provided with a manually operable balloon control element 31. The control element is preferably realized as a depressible control button. The control element 31 is preferably arranged in the vicinity of the pump 5. Preferably, the control element is arranged within 5 cm from the pump.

The control unit further comprises a valve for controlling transfer of irrigation liquid from the reservoir to the probe. The valve may be operated by a control element 32, such as a depressible control button.

In this embodiment, the pump 5 is a manually operable pump. However, other types of pumps are also feasible. In the shown example, the manually operable pump is a bulb pump, comprising an inlet 51, provided with a one-way valve 52, allowing a fluid to enter but not exit the pump. Further, the pump comprises a pumping compartment 53 and outlet 54, provided with a one-way valve 55, allowing a fluid to exit but not enter the pump. The pumping compartment is made of a resilient, squeezable material, which retains it shape when unloaded. By squeezing the pumping compartment, the fluid is pumped out through the outlet, and when the squeezing is relieved, the pumping compartment retains its original shape, thereby sucking in fluid through the inlet.

In the illustrative example, the pump is used to pump air. Thus, the pump 5 pumps air into the balloon 21 for inflation, and the air is releasable through the valve 31.

In this embodiment, the tubing connecting the reservoir, control unit and probe comprises a first tube 61 connecting the reservoir and the probe and a second tube 62 connecting the pump and the probe.

Preferred materials for the bulb pump and the balloon can be any suitable material e.g. such as PVC, latex, TPE or PU. However, other materials providing similar properties can likewise be used.

As an alternative to having a manually operated pump, it is possible to use a powered pump, such as an electrically or pneumatically operated pump.

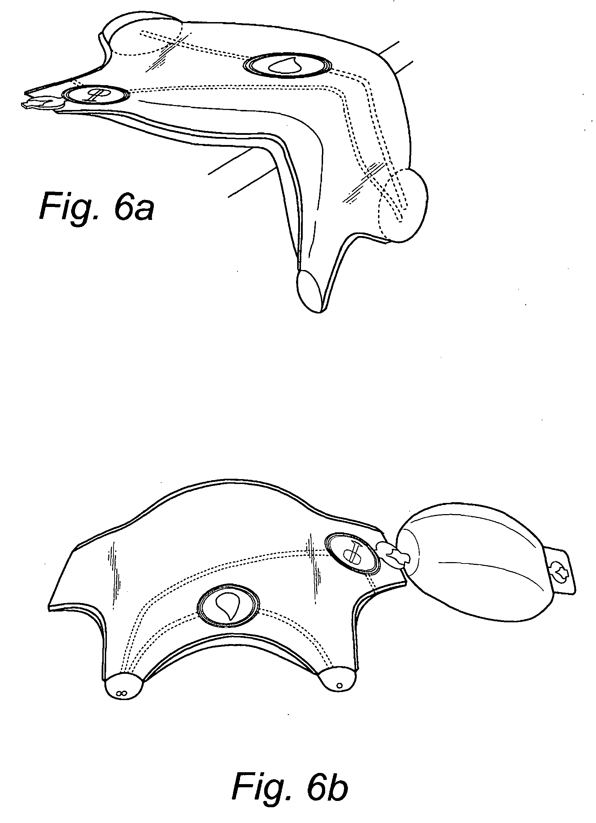

However, the control unit need not be in the form of a shape. Many alternative shapes are also feasible, and may be preferred, depending on how and by whom the irrigation system is to be used. For example, some alternative shapes are illustrated in Figs 6. In Fig 6A, the control unit has an angled shape. In Fig 6B, the control unit has a curved shape. Both these shapes are e.g. advantageous for placing the control unit over a curved or angled support surface. For example, this may be used for placing the control unit over the thigh of the user. The curved or angled shape will make the control unit retain its resting position more efficiently.

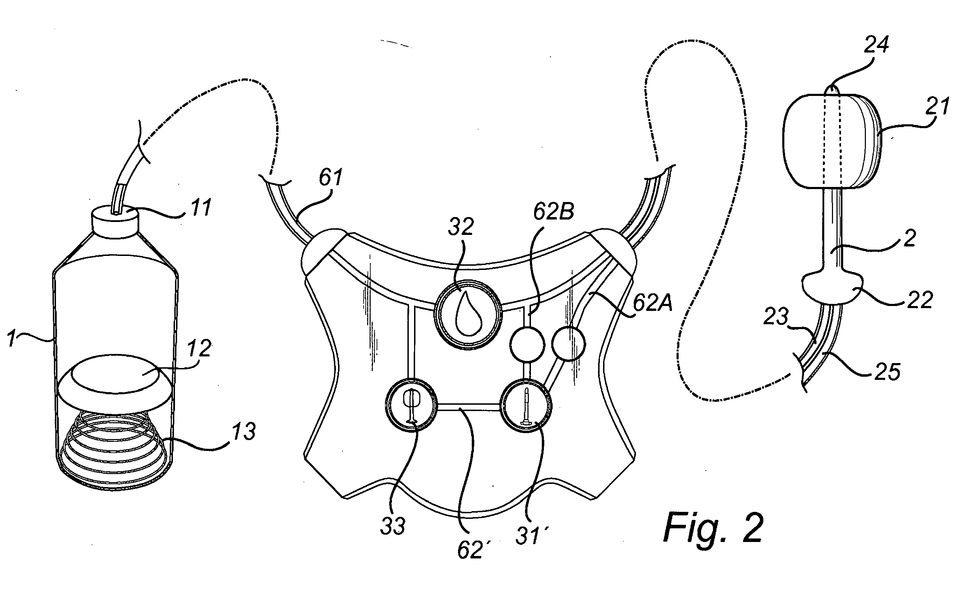

The second embodiment, illustrated in Fig 2, resembles the above-described first embodiment. However, here the balloon 21 is arranged to be filled with irrigation liquid instead of air. To this end, the control unit comprises a valve operable by means of a control element 33, such as a depressible control button, to fill the balloon. Another valve, controlled by means of a control element 31', such as a depressible control button, may be used to release the liquid from the balloon. The liquid may be released through the probe.

The valve 32 is preferably closed by default, and opened up to release irrigation liquid from the reservoir to be discharged through the probe. The valve 33 is also closed by default, and opened up to release irrigation liquid from the reservoir to the balloon. The valve 31' is as a default closed to a tube/channel 62B and opened to a tube/channel 62A. By activation of the valve 31', the tube/channel 62B will be opened, allowing fluid from the balloon to be discharged back through tube 62A, out through tube 62B, and further into tube 61 for discharge through the probe.

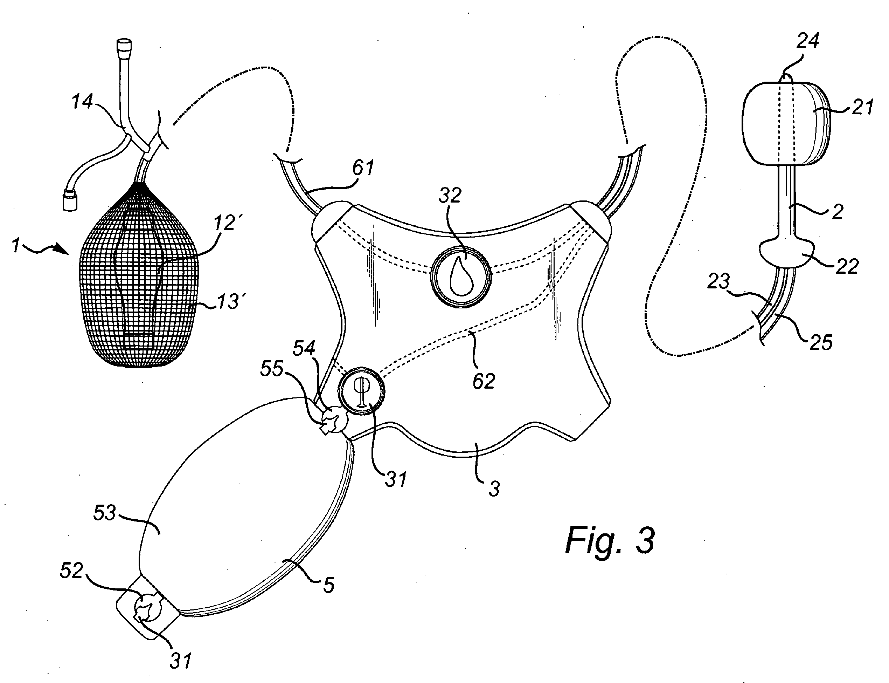

The third embodiment, illustrated in Fig 3, also resembles the above-discussed previous embodiments. However, here the reservoir comprises an elastically stretchable container 12', which when elastically stretched exerts a mechanical pressure on the irrigation liquid therein. Preferably, the reservoir may hereby form an elastically stretchable balloon, which is inflated by the irrigation liquid, and thereby provides a pressure to expel the irrigation liquid when the control valve is opened. To prevent overfilling of the reservoir, the reservoir may further comprise an outer container 13 of a non-stretchable material. Hereby, the outer container forms an outer boundary for the elastically stretchable container. The non-stretchable outer container may be formed as a rigid outer container, or as a flexible outer container. For example, a flexible outer container may be formed as a net of a non-stretchable material, as in the exemplary embodiment of Fig 3.

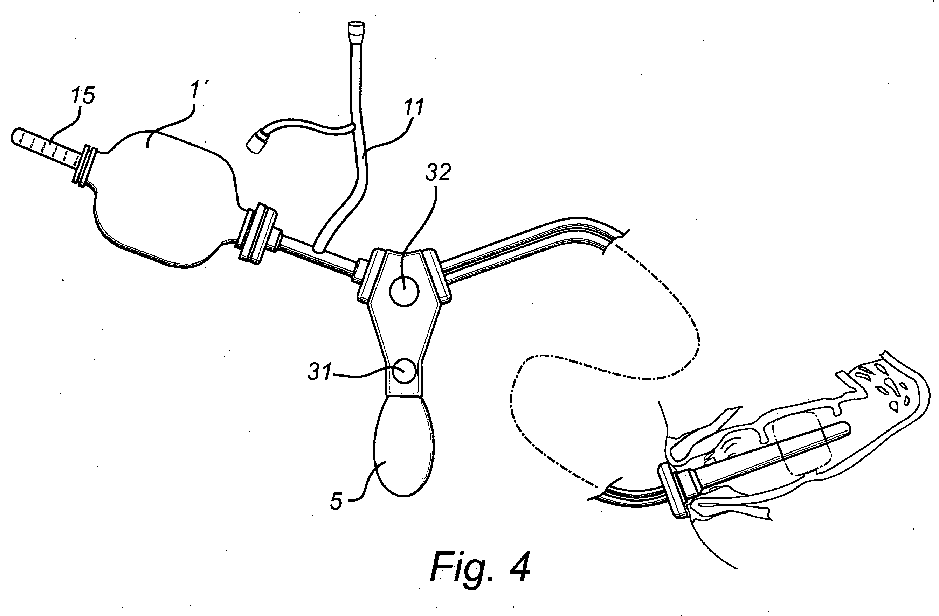

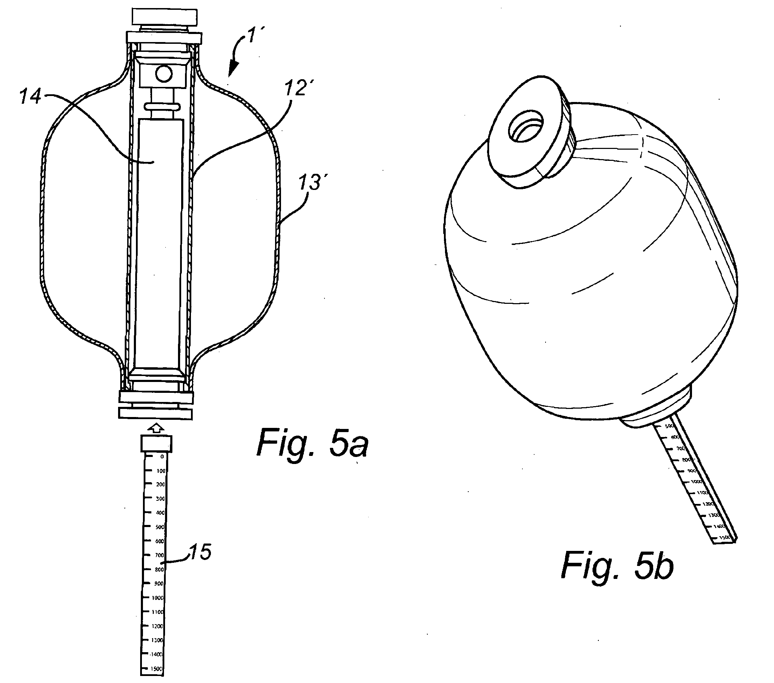

If an release or overload valve is provided on the reservoir, the non-stretchable outer container is redundant, and may be dispensed with. The fourth embodiment, illustrated in Figs 4 and 5, also resembles the above-discussed previous embodiments. However, here the reservoir also comprises an elastically stretchable container 12', which when elastically stretched exerts a mechanical pressure on the irrigation liquid therein, as in the previously discussed embodiment. To prevent overfilling of the reservoir, the reservoir may further comprise an outer container 13' of a non-stretchable material. In this embodiment, the outer container 13' is formed as a rigid or semi-rigid outer container.

The irrigation system further preferably comprises a volume indicator, as best seen in Fig 5, arranged within the reservoir. This enables the user to precisely monitor and control the dose of irrigation liquid being used. According to the illustrative embodiment, the volume indicator comprises two telescopically connected parts 14, and a measuring element 15 connected to one of said parts and extending out through the other of said parts. The telescopically connected parts preferably extend through the reservoir, and each part being connected to one wall of the reservoir. The measuring element is preferably connected to a rearward telescopic part or a rearward wall, and extends out through a forward telescopic part or a forward wall. Hereby, when the distance between the walls changes due to filling of the reservoir, this is translated into a movement of the measuring element. The measuring element 15 may have a scale, indicating the volume of irrigation fluid remaining in the reservoir and/or the volume of irrigation fluid that has been discharged after filling. Additionally or alternatively, the marking may comprise indications of whether a sufficient volume of irrigation liquid has been discharged, such as a green area indicating a correct dose.

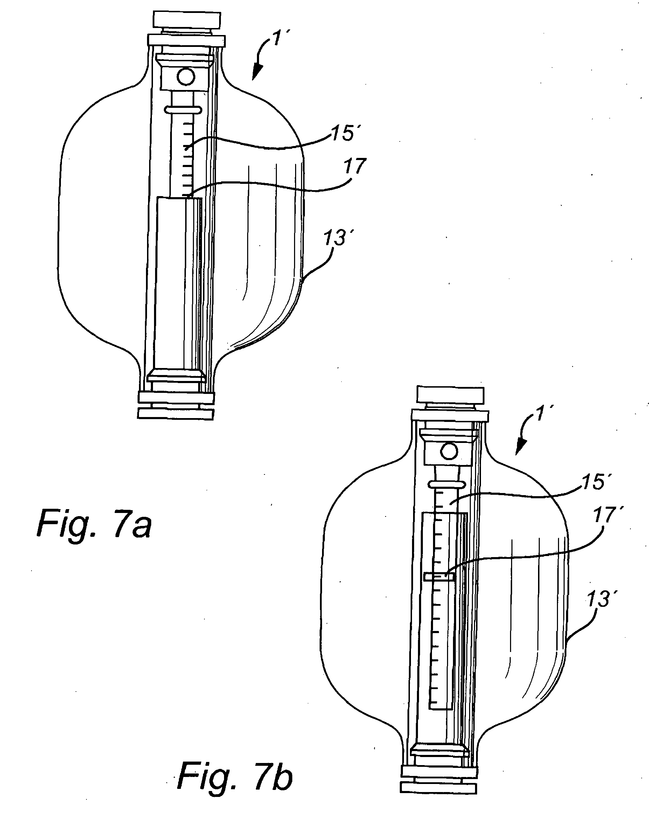

Alternatively, as illustrated in Fig 7A, the measuring scale or measuring indications 15' may be arranged on the inner telescopic part, whereby any separate measuring element becomes redundant. This alternative is particularly useful when the reservoir is made wholly or partially of transparent material. The reading on the scale may be taken at the interjunction between the two telescopic parts, i.e. by using the open end 17 of the outer telescopic part as a marker.

In yet another alternative, illustrated in Fig 7B, the outer telescopic part is also at least partly transparent. Hereby, the measuring scale 15' is readable through the outer telescopic part, and a reading marker 17' or the like may be arranged on the surface of the outer telescopic part.

It is also possible to arrange the measuring scale or measuring indication on the outer telescopic part, and to have the marker on the inner telescopic part.

The telescopic parts may also be operatively connected to a release or overload valve. Hereby, the valve may be automatically opened when a certain extension level between the telescopic parts has been reached, thereby preventing overfilling of the reservoir. The released liquid may be returned by a return tube to the sink. Further, the control unit, comprising the control elements 31 and 32, is here realized as a narrow and slim control element, which is easily gripped by the user.

The irrigation liquid can be any liquid which is capable of irrigation the body cavity of interest. In order to stimulate bowel movements suitable irrigation liquids includes water, hypertonic aqueous salt solutions, solutions or suspensions of cathartic agents, such as bisacodyl or phenolphthalein, and mineral oil.

As discussed above, the reservoir may be loaded by the pressure from the water distribution system, by connecting the reservoir to a water hose. Connection to the water tap may e.g. be achieved by a threaded connector or the like. Further, an overpressure release valve may be used to prevent overfilling/overpressurization of the reservoir. However, in case the water pressure in the water distribution system is insufficient, or if filling of the reservoir occurs at a location where there is no access to the water distribution system, pressurization of the reservoir may be obtained in other ways. For example, the springs may be pre-loaded by some mechanical compression means, etc. Further, the reservoir may be pressurized/pre-loaded by means of a pump, such as a manually operated pump or a powered pump, by means of a gas cartridge, or the like.

By use of the present invention, anal irrigation can be carried out by the following steps:
- The reservoir is filled with a liquid, e.g. regular tap water, whereby a pre-loaded, pressurized state is obtained,
- The reservoir and the probe are connected to the control unit,
- The probe is inserted into the rectum,
- The pump 5 is operated to inflate the balloon, for fixing the probe in the body, or alternatively, the control element for filling the balloon with irrigation liquid is activated,
- The control element 32 is activated to transfer irrigation liquid from the reservoir to the probe for irrigation,
- When irrigation is completed, the balloon is deflated, and
- The probe is removed.

The person skilled in the art realizes that the present invention is not limited to the preferred embodiment. For example many different types of hand-operated or powered pumps may be used. Further, the valves and the controls for the valves may be realized in many different ways. Also, the pumping of the irrigation liquid may be direct or indirect. Further, the mechanical pressurization of the reservoir may be achieved by many different types of springs, elastic means and the like. The valves above have been discussed as being either opened or closed. However, the valves may also be of a type allowing setting of intermediate opening states, in a stepped or continuous fashion.

Such and other obvious modifications must be considered to be within the scope of the present invention, as it is defined by the appended claims. It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting to the claim. The word "comprising" does not exclude the presence of other elements or steps than those listed in the claim. The word "a" or "an" preceding an element does not exclude the presence of a plurality of such elements. Further, a single unit may perform the functions of several means recited in the claims.

## Claims

1. An irrigation system comprising:
a reservoir (1, 1') for an irrigating liquid;
a probe (2) for arrangement in a user;
a control unit (3) for controlling the transfer of said irrigation liquid; and
tubing providing fluid communication between said reservoir, control unit and probe;
**characterized in that** said reservoir (1, 1') is arranged to exert a mechanical pressure on the irrigation liquid therein, thereby maintaining the irrigation liquid under pressure, and **in that** said control unit (3) comprises a control valve (32) for releasing irrigation liquid from said reservoir (1, 1') for discharge through said probe (2).

2. The irrigation system of claim 1, wherein the reservoir (1') comprises an elastically stretchable container (12'), which when elastically stretched exerts a mechanical pressure on the irrigation liquid therein.

3. The irrigation system of claim 2, wherein the elastically stretchable container (12') forms an inflatable balloon.

4. The irrigation system of claim 2 or 3, wherein the reservoir further comprises an outer container (13') of a non-stretchable material, said outer container forming an outer boundary for the elastically stretchable container (12').

5. The Irrigation system of claim 1, wherein the reservoir (1) is formed of a rigid or semi-rigid material, and having at least one moveable wall (12) and a retention element (13) to exert a force to retain the moveable wall in a contracted position.

6. The irrigation system of claim 5, wherein the retention element (13) is a spring, and preferably a coil spring.

7. The irrigation system of any one of the preceding claims, wherein the probe (2) comprises an inflatable balloon (21) for fixing the catheter in a body cavity, and wherein the control unit (3) is further arranged to control the fluid for said balloon.

8. The irrigation system of claim 7, wherein the control unit (3) further comprises a second control valve (33) for releasing irrigation liquid from said reservoir for inflation of the inflatable balloon.

9. The irrigation system of claim 7, further comprising a pump (5) arranged for pumping a fluid to the balloon for inflation.

10. The Irrigation system of claim 9, wherein the pump (5) is a manually operable pump, such as a bulb or a bellow pump.

11. The irrigation system of any one of the claims 8-10, wherein the control unit further comprises a third control valve (31, 31') for releasing fluid from the balloon when inflated.

12. The irrigation system of any one of the preceding claims, further comprising a volume indicator arranged within the reservoir.

13. The irrigation system of claim 12, wherein the volume Indicator comprises two telescopically connected parts (14, 15), and a measuring scale, measuring indication arranged on one of said parts, or a measuring element connected to one of said parts and extending out through the other of said parts.

14. The irrigation system of any one of the preceding claims, wherein the control unit (3) has a curved or angled shape, with tubing extending therethrough.

## Patentansprüche

1. Irrigationssystem, umfassend:
ein Reservoir (1, 1') für eine Irrigationsflüssigkeit;
eine Sonde (2) zur Anordnung in einem Benutzer;
eine Steuereinheit (3) zum Steuern der Übertragung der Irrigationsflüssigkeit; und
Schlauchmaterial, das eine Fluidverbindung zwischen dem Reservoir, der Steuereinheit und der Sonde vorsieht;
**dadurch gekennzeichnet, dass** das Reservoir (1, 1') eingerichtet ist, einen mechanischen Druck auf die Irrigationsflüssigkeit darin auszuüben, wodurch die Irrigationsflüssigkeit unter Druck gehalten wird und dadurch, dass die Steuereinheit (3) ein Steuerventil (32) zum Freisetzen der Irrigationsflüssigkeit aus dem Reservoir (1, 1') zur Abgabe durch die Sonde (2) aufweist.

2. Irrigationssystem nach Anspruch 1, wobei das Reservoir (1') einen elastisch dehnbaren Behälter (12') aufweist, der, wenn er elastisch gedehnt ist, einen mechanischen Druck auf die Irrigationsflüssigkeit darin ausübt.

3. Irrigationssystem nach Anspruch 2, wobei der elastisch dehnbare Behälter (12') einen aufblasbaren Ballon bildet.

4. Irrigationssystem nach Anspruch 2 oder 3, wobei das Reservoir ferner einen äußeren Behälter (13') aus einem nicht dehnbaren Material aufweist, wobei der äußere Behälter eine äußere Begrenzung für den elastisch dehnbaren Behälter (12') bildet.

5. Irrigationssystem nach Anspruch 1, wobei das Reservoir (1) aus einem steifen oder halbsteifen Material gebildet ist und mindestens eine bewegliche Wand (12) und ein Halteelement (13) zum Ausüben einer Kraft aufweist, um die bewegliche Wand in einer zusammengezogenen Position zu halten.

6. Irrigationssystem nach Anspruch 5, wobei das Halteelement (13) eine Feder ist und vorzugsweise eine Schraubenfeder ist.

7. Irrigationssystem nach einem der vorhergehenden Ansprüche, wobei die Sonde (2) einen aufblasbaren Ballon (21) zum Fixieren des Katheters in einer Körperhöhle aufweist und wobei die Steuereinheit (3) ferner eingerichtet ist, um das Fluid für den Ballon zu steuern.

8. Irrigationssystem nach Anspruch 7, wobei die Steuereinheit (3) ferner ein zweites Steuerventil (33) zum Freisetzen der Irrigationsflüssigkeit aus dem Reservoir zum Aufblasen des aufblasbaren Ballons aufweist.

9. Irrigationssystem nach Anspruch 7, das ferner eine Pumpe (5) aufweist, die für das Pumpen eines Fluids zu dem Ballon zum Aufblasen eingerichtet ist.

10. Irrigationssystem nach Anspruch 9, wobei die Pumpe (5) eine manuell zu betätigende Pumpe, beispielsweise eine Ballonpumpe oder eine Balgpumpe, ist.

11. Irrigationssystem nach einem der Ansprüche 8 - 10, wobei die Steuereinheit ferner ein drittes Steuerventil (31, 31') zum Freisetzen von Fluid aus dem Ballon im aufgeblasenen Zustand aufweist.

12. Irrigationssystem nach einem der vorhergehenden Ansprüche, ferner umfassend eine Volumenanzeige, die innerhalb des Reservoirs angeordnet ist.

13. Irrigationssystem nach Anspruch 12, wobei die Volumenanzeige zwei teleskopartig verbundene Teilstücke (14, 15) und eine Messskala aufweist, wobei die Messanzeige auf einem der Teilstücke angeordnet ist, oder ein Messelement, das mit einem der Teilstücke verbunden ist und sich durch das andere der genannten Teile heraus erstreckt.

14. Irrigationssystem nach einem der vorhergehenden Ansprüche, wobei die Steuereinheit (3) eine gekrümmte oder abgewinkelte Form, mit einem Schlauch, der sich dort hindurch erstreckt, aufweist.

## Revendications

1. Système d'irrigation comprenant :
un réservoir (1, 1') pour un liquide d'irrigation ;
une sonde (2) à disposer dans un utilisateur ;
une unité de commande (3) pour commander le transfert dudit liquide d'irrigation ; et
une canalisation assurant une communication de fluide entre ledit réservoir, l'unité de commande et la sonde ;
**caractérisé en ce que** ledit réservoir (1, 1') est conçu pour exercer une pression mécanique sur le liquide d'irrigation qu'il contient, en maintenant ainsi le liquide d'irrigation sous pression, et **en ce que** ladite unité de commande (3) comprend une vanne de commande (32) pour délivrer du liquide d'irrigation depuis ledit réservoir (1,1') pour déchargement par l'intermédiaire de ladite sonde (2).

2. Système d'irrigation selon la revendication 1, dans lequel le réservoir (1') comprend un contenant élastiquement extensible (12') qui, une fois étiré élastiquement, exerce une pression mécanique sur le liquide d'irrigation contenu dedans.

3. Système d'irrigation selon la revendication 2, dans lequel le contenant élastiquement extensible (12') forme un ballon gonflable.

4. Système d'irrigation selon la revendication 2 ou 3. dans lequel le réservoir comprend en outre un contenant extérieur (13') en matériau non extensible, ledit contenant extérieur formant une limite extérieure du contenant élastiquement extensible (12').

5. Système d'irrigation selon la revendication 1, dans lequel le réservoir (1) est formé d'un matériau rigide ou semi rigide et comporte au moins une paroi mobile (12) et un élément de retenue (13) pour exercer une force afin de retenir la paroi mobile en position contractée.

6. Système d'irrigation selon la revendication 5, dans lequel l'élément de retenue (13) est un ressort et de préférence un ressort hélicoïdal.

7. Système d'irrigation selon l'une quelconque des revendications précédentes, dans lequel la sonde (2) comprend un ballon gonflable (21) pour fixer le cathéter dans une cavité corporelle et dans lequel l'unité de commande (3) est en outre conçue pour contrôler le fluide destiné audit ballon.

8. Système d'irrigation selon la revendication 7, dans lequel l'unité de commande (3) comprend en outre une seconde vanne de commande (33) pour délivrer du liquide d'irrigation depuis ledit réservoir pour gonfler le ballon gonflable.

9. Système d'irrigation selon la revendication 7, comprenant en outre une pompe (5) conçue pour pomper un fluide dans le ballon pour le gonfler.

10. Système d'irrigation selon la revendication 9, dans lequel la pompe (5) est une pompe actionnable manuellement, comme une pompe à poire ou à soufflet.

11. Système d'irrigation selon l'une quelconque des revendications 8 à 10, dans lequel l'unité de commande comprend une troisième vanne de commande (31, 31') pour délivrer du fluide depuis le ballon une fois qu'il est gonflé.

12. Système d'irrigation selon l'une quelconque des revendications précédentes, comprenant en outre un indicateur de volume disposé dans le réservoir.

13. Système d'irrigation selon la revendication 12, dans lequel l'indicateur de volume comprend deux pièces connectées de manière télescopique (14, 15) et une échelle de mesure, un indicateur de mesure disposé sur une desdites pièces ou un élément de mesure connecté à une desdites pièces et s'étendant à travers l'autre desdites pièces .

14. Système d'irrigation selon l'une quelconque des revendications précédentes, dans lequel l'unité de commande (3) a une forme courbée ou angulaire avec une canalisation s'étendant à travers.
